# EUROPEAN PATENT SPECIFICATION

(11) **EP 2 373 730 B1**
(45) Date of publication and mention of the grant of the patent: **08.05.2013**
(21) Application number: 09768306.4
(22) Date of filing: 09.12.2009
(51) Int. Cl.: C08K 13/02

(54) **COMPACTED PELLETIZED ADDITIVE BLENDS FOR POLYMERS**
KOMPAKTIERTE PELLETIERTE ADDITIVMISCHUNGEN FÜR POLYMERE
MÉLANGES D'ADDITIFS GRANULÉS ET COMPACTÉS POUR POLYMÈRES

(30) Priority: 12.12.2008 US 122247 P
(43) Date of publication of application: 12.10.2011
(73) Proprietor: Ingenia Polymers, Inc., Houston, TX 77027 (US)
(72) Inventor: CHATTERJEE, Ananda, M., Missouri City TX 77459 (US); LAFRANCE, Tania, M., Lago Vista TX 78645 (US); D'UVA, Salvatore, Brantford ON N3T 6J1 (CA)
(74) Representative: Gates, Marie Christina Esther
(86) International application number: PCT/US2009/067315
(87) International publication number: WO 2010/068666

(56) References cited:
- US-A- 5 053 444

## Description

This application claims priority to U.S. Provisional Patent Application Serial Number 61/122,247, entitled "COMPACTED PELLETIZED ADDITIVE BLENDS FOR POLYMERS" filed on December 12, 2008.

The invention relates generally to the field of polymer additives and specifically to compacted additive blends, or polymer stabilization agent blends, which can be added in post-polymerization processes to enhance the processability and property performance of polymers.

Additives and additive blends are typically used to protect polymers from thermo-oxidative degradation, to provide long-term resistance to heat or light (including ultraviolet), to neutralize residual catalyst and to enhance various performance properties of the finished product. Polymer additives typically come in liquid, powder, granule, bead, pastille, or pellet form. These additives can be added to the polymer during post-reactor extrusion operations. Numerous techniques may be employed to introduce the additives to the polymer in the process stream. In solution, suspension or slurry phase polymerization processes, additives and additive blends are added to a liquid before being introduced to the post-reactor polymer-liquid slurry. Alternatively, the additives can be added to the final melt stream of polymer via a side arm extruder or other device which can melt the additives and introduce them to the polymer stream. In this case, there will typically be further mixing via an extruder or other mixing device and pumping of the polymer/additive mixture through a die for pelletizing the final polymer resin. In other polymerization processes such as those using a gas phase reactor, the polymer exits the reactor as a powdered "reactor granule." In this case, additives can be added to the polymer in several different ways. The additives can be added to the solid "reactor granule" powder stream. This can be packaged off as a final saleable product or it can be further fed to an extruder or other melting device in order to mix and homogenize the polymer and disperse the additives into the molten polymer. When additives are added to the solid "reactor granule" powder stream, the additives can be introduced at this stage via their neat forms, typically powder, or via a concentrate or masterbatch form. This mixture is subsequently pumped through a die for pelletization. Alternatively, in this type of process, the additives can be introduced via a side arm extruder. The side arm extruder melts the additives and feeds them into a molten polymer stream where they are further mixed into the final polymers and pelletized. In all of these techniques, the addition of the additives in powder form can be difficult in terms of handling and feeding, and in the case of some additives, they can pose a potential health, fire, and explosion risk. If the polymer system requires the addition of several components, the additives must be either pre-blended, or the use of multiple feeders is required. When a side-arm extruder is used, it is not common to feed the powdered additives directly for many reasons. In addition to the above mentioned issues with handling and feeding the additives in powder form to the extruder, the melting and viscosity behavior of the additives and the additive mixtures are typically not suitable for direct addition via this method.

Preparation of non-dusting pellet forms of additive blends solves many of these problems. Among these processes, extrusion of the 100% or all-additive blend to form pellets has been taught in the patent art. U.S. Patent No. 5,240,642 entitled "Process for Obtaining Granular Forms of Additives for Organic Polymers" describes a process for making low-dust granules of an additive blend including a phenolic antioxidant and an acid neutralizer, using an extruder. U.S. Patent No. 5,844,042 entitled "Process for Obtaining Granular Forms of Additives for Organic Polymers" describes granular forms of additive blends prepared by extrusion process. The additive blend is forced through a die to form strands and then said strands are cut to form pellets.

U.S. Patent No. 5,597,857, entitled "Low-Dust Granules of Plastic Additives" describes extruded additive blend pellets comprising 10-100% calcium stearate. Calcium stearate is at least 80% melted, and the blend is forced through a die, face cut (water ring or underwater), and then cooled.

U.S. Patent No. 6,740,694B2 entitled "Preparation of Low-Dust Stabilizers" describes extrusion of additive blends containing amorphous form of additives. The additive blend is melted and subcooled, thereby forming a high viscosity strand which is pelletized.

While the above processes involve extrusion of additive blends, another technology has been described for compaction of additives into pellets. U.S. Patent No. 5,846,656 (Ciba Specialty Chemicals, Basel, Switzerland) covers a pellet mill type compaction process where 2-50% of a 'melt preventing compound' (binding agent) is added to a stabilizer or additive system to prevent melting of the stabilizer. The relatively low melting binder can be hindered phenolic antioxidant (AO) like Irganox® 1076 (Ciba), calcium stearate, glyceryl mono-stearate (GMS), oleamide, stearic acid, palmitic acid, low density polyethylene (LDPE) etc.

U.S. Patent No. 6,515,052 (Albemarle Corporation, Baton Rouge, LA) entitled "Granular Polymer Additives and Their Preparation" describes using a solvent in a compaction process to improve the yield and quality (lower friability) of a compacted additive blend including a phosphite stabilizer. A solvent (e.g. alcohol, hexane) is added to the dry additive blend and subsequently the mixture is processed through the pellet mill. The solvent partially solvates the phosphite. After passing through the pellet mill, the solvent is removed. This gives a very high yield since a wet paste is being extruded.

U.S. Patent No. 6,800,228 (Albemarle) entitled "Sterically Hindered Phenol Antioxidant Granules Having Balanced Hardness" describes using a solvent for the preparation of compacted additive blends including a phenolic AO. This patent extends the technology taught in 6,515,052 to phenolic antioxidants. The solvent is usually alcohol, cyclohexane or a mixture thereof; adjusting the amount and ratio of solvents can control the hardness and friability of the pellets produced.

U.S. Patent No. 6,596,198, (Albemarle) broadly teaches compacted blends of 5-50% stabilizer with a low melting binder, for example oleamide (lubricant or mold release agent), Irganox® 1076 (Ciba), glycerol, GMS (e.g. Pationic® 1042, Caravan Ingredients, Kansas City, Missouri), and others. This patent also teaches an extrusion process for preparing additive blends.

U.S. Patent No. 6,033,600 (General Electric Company, Fairfield, Connecticut) describes compacted pellet blends, typically a mixture of 10-90% penta-erythritol diphosphite, 10-90% phenolic antioxidant, 2-60% metallic stearate and 1-10% hydrotalcite. It is also taught that pre-compacting (such as in a roll mill) before the pellet mill operation significantly increases yield of pellets.

The references described above provide for low-dusting forms of additive blends that can be more conveniently and accurately fed to post-reactor extrusion operations for addition to a polymer. Such solid additive blends can be added directly to a polymer stream into an extruder or other melting device whereby the polymer is melted and the additives are then blended into the molten polymer, which is subsequently pelletized.

### SUMMARY

The current invention pertains to compacted additive blends in which high melting or non-melting metallic silicate or other additives are used as a compaction aid, to increase the resistance to friability of the compacted pellets. In addition to this benefit, the compaction aid of this invention can also minimize melting tendency of the additives during the compaction process in a mill.

The current invention improves the friability or attrition performance of pellets produced by compaction in a pellet mill, via the use of solid non-melting binding aids. Friability and attrition usually refer to the same phenomenon, wherein the pellets can generate fine powder inside its container during transport of the pellets. The resistance to friability or attrition is highly desirable, as very little fines are generated. This is referred to as high attrition performance. The friability is evaluated in the laboratory by attrition testing, in which steel balls are used to impact the pellets while being shaken inside a sieve stack. The traditional binding agents used in additive compaction process are relatively lower melting organic additives, e.g. oleamide, glyceryl monostearate, Irganox 1076 (Ciba), etc., as described in US Patent No. 5,846,656. It has been discovered, surprisingly, that solid non-melting non-migrating additives, including minerals, can aid the compaction process such that improved attrition performance (lower generation of fines) can be achieved.

The word "non-melting" in this invention means that the high-melting solid binding or compaction aid does not melt into a liquid in the environment of the compaction mill, that is, under the operating temperature and other conditions of the compaction process. The compaction aid of this invention typically has a melting point above 300°C. This is in contrast to the prior art (e.g. Ciba 5,846,656 patent cited above), where a low-melting solid acts as a binding agent; the binding agent melts during compaction and helps to bond the other components together into a pellet particle. The word 'non-migratory' in this invention means that the solid compaction aid of the invention does not diffuse from one point to another within the additive pellet. There is essentially no mobility of the solid binding aid inside the compacted additive pellet. In contrast, the low-melting binding agents of prior art can melt during compaction and the resulting liquid can migrate or diffuse in space to a different position of lower (including zero) concentration inside the additive pellet. The invention is further differentiated from, and improved over, the prior art due to the fact that the pelletization and high attrition performance of this invention are achieved without the use of any solvent or pre-compaction technique. This invention is useful as it will allow better utilization of commercial compacted additive pellets, because the pellet with reduced attrition or friability can be transported to the destination with less generation of fines. Such solid binding aid technology for attrition improvement was not available before this invention. Pellets with undesirable high attrition arrive at the user's facility with significant level of fine powder. The latter is objectionable (e.g. due to dust exposure to operators, explosivity issues, etc.) and often represents loss of usable product.

The high-melting or non-melting types of additives used in the current invention have no deleterious effect on the final end use, as they are essentially inert and non-migratory after they are introduced into the polymer resin. This invention is surprising in view of the non-melting, non-migratory solid particulate nature of the compaction aid. In previously-used compaction processes a low melting material is generally used as binder. Typical examples are octadecyl-3- (3,5-di-tertiary-butyl-4-hydroxyphenyl) propionate (sold commercially as Irganox 1076 by Ciba, or Songnox 1076 by Songwon, etc.), glyceryl monostearate ("GMS"), oleamide, erucamide, and others. These additives melt during the compaction process to wet out the other additives and bind them together to form a compacted mass of additive blend. High melting materials have been regarded as unsuitable as binders in the prior art.

A typical commercial compacted additive blend often shows high friability when the blend is impacted by solid attriting particles or conveyed in manufacturing and handling processes. Under such conditions the compacted material generates fines. In the present invention, new and novel additive blend compositions of matter (sometimes referred to as "Superblend") are provided, wherein the friability of the compacted additive pellets is reduced by the addition of a non-melting, non-migratory component, including metallic silicate and silica particles. As a result a much lower level of fine powder is generated from the compacted additive blends (compared to other known blends), thereby improving the economics of the manufacturing and pellet conveying processes.

The compacted pelletized additive blend consists of a mixture of two or more additives totaling 100% additives, that is, no polymer carrier is present. The compaction aids in this invention are generally high melting metal silicate or silica type materials. The ranges of compaction aids in the compacted palletized additive blend can be in the range of from about 1 wt. % to about 30 wt. %, preferably from about 3 wt. % to about 20 wt. %, and most preferably from about 5 wt. % to about 10 wt. %. "Weight %" as used herein denotes % in weight of the fine compacted palletized additive blend. The base additives used in the present invention are generally crystalline additives having a peak melting temperature (or additive melting temperature), or amorphous additives having a glass transition temperature (or additive glass transition temperature) within the range of normal processing temperatures of polyolefins or other polymers. The ranges of base additives in the compacted palletized additive blend can be in the range from about 70 wt. % to about 99 wt. %, preferably from about 80 wt. % to about 97 wt. %, and most preferably from about 90 wt. % to about 95 wt. %. These compacted additive blends are useful in polymer resin production processes, especially in the manufacture of polymers whereby after polymerization, the polymer is fed to an extruder or other device in which the polymer is melted in order to introduce additives to the molten polymer stream. Such additives are essential for maintaining and improving the properties of polymers and for adding functionality or other performance features to said polymers.

Using the techniques of the present invention, blends of neat additives can be made which are dust-free, and robust in that they are easily conveyed using pneumatic air conveying. Such blends are easily fed to an extruder or other device wherein they are introduced into a molten polymer stream. In this step, the additive blend is diluted to the final end-use level for stabilization or introduction of appropriate additive functionality to the polymer resin being produced. Such 100% additive blends can also be useful when fed directly to the solid polymer and physically blended with the base polymer prior to the final melting, mixing, and pelletizing, or simultaneously fed to the final melting, mixing, and pelletizing device for the polymer resin being produced. The blend of neat additives thus produced, with reduced friability, allow for significant cost savings in various polymer resin manufacturing processes.

### DETAILED DESCRIPTION

The particulars of the invention shown herein are by way of example only. They are meant to illustrate various embodiments of the invention and not meant to limit the principles or concepts of the invention.

Given below are the condensed and shortened (and by no means exhaustive) customary definitions, known in the art, of certain terms whose condensed definitions may aid in the description of the invention.

"Base polymer": the polymer which is to be stabilized, colored, functionalized, or otherwise modified by the additive or blend of additives.

"Ingenia Superblend^{™}" or "Superblend^{™}:" a 100% or neat additive blend or concentrate of additives, fillers, colorants, and other components which is then blended into the base polymer to be stabilized, colored or modified, rather than adding the additive, filler or colorant separately. Examples of the additives include hindered phenolic antioxidants (such as Irganox 1010, 1076, Ethanox 330); phosphite antioxidants (such as Irgafos 168, Ultranox 626); phosphonite antioxidants (such as Sandostab P-EPQ); acid neutralizers (such as calcium stearate, hydrotalcite DHT-4A which is magnesium aluminum hydroxy-carbonate hydrate); ultraviolet (UV) light stabilizers (such as Chimassorb 944, Tinuvin 622, Cyasorb UV 2908), nucleating agents (such as sodium benzoate); clarifying agents such as 1,3:2,4-bis (3,4- dimethyl benzylidene sorbitol, sold as Millad 3988 by Milliken Chemical; antistatic agents (such as glyceryl mono-stearate; ethoxylated amines); slip agents (such as erucamide, oleamide, behenamide); antiblock agents (such as silica); and fillers (like calcium carbonate, talc, wollastonite). "Ingenia Superblend^{™}" or "Superblend^{™}" is a pelletized or pastillated solid additive blend or mixture, and may be: a) polymer masterbatch which is highly loaded with additives (e.g. 50-90% by weight); b) pastillated, and produced in equipment such as a rotoformer; c) compacted palletized additive blend; and d) melt-extruded pellet of additive blend.

"Binding aid" or "binder:" a low melting or liquid additive, which can facilitate additive binding during the milling process of compacting and pelletizing of additive blends in conventional art.

"Melting point": the peak melting temperature of a crystalline or semicrystalline polymer or crystalline polymer additive as measured by differential scanning calorimetry ("DSC") or other instrument.

"Polymer resin": the final formulation resulting from the combination of the base polymer and additive or additives.

"Softening point": the glass transition temperature of an amorphous additive as measured by differential scanning calorimetry ("DSC") or other instrument.

The pelletized carrier-free 100% additive blend or concentrate, or Superblend, of the present invention is composed of two or more components. The components are a variety of additives characterized by a melting or softening point between 20°C and <300°C and more preferably between 40°- 200°C.

The additive blend is made up of a base additive mixture and one or more compaction aids. The compaction aids include one or more mineral or inorganic high melting additives, present at a low concentration (typically <25%) chosen from metallic silicates, montmorillonite nano-clay, silica, calcium carbonate, barium sulfate, titanium dioxide, zinc oxide, or others, including mixtures of any of these. The Superblend is useful in polymer resin production, especially in manufacturing of polymers whereby after polymerization, the polymer is fed to an extruder or other device in which the polymer is molten in order to introduce additives to the polymer stream.

Unless otherwise specified, additive concentrations in this specification refer to weight percent ("wt. %"). Wt. % of a component is calculated by dividing the mass of the component by the total mass of all of the ingredients in the mixture, expressed as percentage. For example, in a pelletized additive blend containing 20 grams of additive A and 80 grams of other additives, the wt. % of the additive A would be 20%.

Preferred base polymers of the present invention include polyolefins, such as various types of polyethylene, polypropylene, ethylene-propylene copolymers, ethylene-alphaolefin copolymers, polybutene-1, polystyrene, ethylene-vinyl acetate copolymers, ethylene vinyl alcohol copolymers, styrene-butadiene copolymers, and blends thereof; and copolymers derived from monomers mentioned above.

The additives present at 100% total concentration (with no carrier polymer) in the blend of the present invention include additives known to those skilled in the art such as phenolic antioxidants, processing stabilizers, acid neutralizers, ultraviolet ("UV") light stabilizers including UV light absorbers, antistatic agents, metal deactivators, slip agents, antiblock agents, nucleating agents, lubricants and mold release agents, flame retardants and others, including mixtures of any of these. Particular examples of such additives include hindered phenolic antioxidants, phosphite and phosphonite stabilizers, hindered amines, metallic (e.g. calcium, zinc) stearates, triazines, benzophenones, benzotriazoles, hydroxybenzoates, benzofuranones including 3-(4-(2-acetoxyethoxy)phenyl )-5,7-di-tertiary-butyl-benzofuran-2-one, di(rape oil) alkyl-N-methyl amine oxide, possessing a melting or softening point in the range of about 20° to about <300°C and more specifically in the range of about 40° to about 200°C. In preferred embodiments, the base additive mixture has a melting temperature less than 90°C, as measured by differential scanning calorimetry or other suitable instrument. Some examples of additives useful in this invention are as follows.

Hindered phenols are known as primary antioxidants for plastics and contain one or more groups of the formula given below: 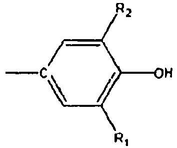 where R₁ and R₂ are methyl, tertiary-butyl, unsubstituted alkyls, or substituted alkyls.

Hindered phenols, useful as one or more of the antioxidant additives in the present invention, should have a melting or softening point in the range of about 50°C to about 300°C, more preferably in the range of about 100°C to about 250°C. Hindered phenols particularly useful in the present invention include, but are not limited to:
(a) AO-10, or penta-erythritol tetrakis (3-(3,5-di-t-butyl-4-hydroxyphenol)propionate), e.g. Evernox 10, Irganox 1010, Anox 20, etc., having the structure below: 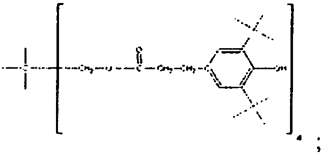
(b) AO-1790, or 1,3,5-tris(4-tert-butyl-3-hydroxy-2,6-dimethylbenzyl)-1,3,5-triazine-2,4,6-(1H,3H,5H)-trione, having the structure below: 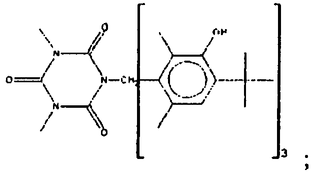
(c) AO-129, or 2,2'-ethlidenebis (4,6-di-tert-butylphenol), having the structure below: 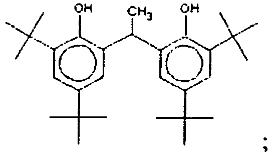
(d) AO-702, or 4,4'-methylenebis(2.6-di-tertiary-butylphenol), having the structure below: 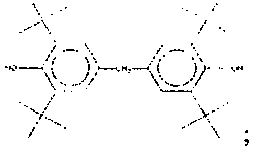 or
(e) AO-246, or 2,4,6-tri-tert-butyl-phenol, having the structure below:

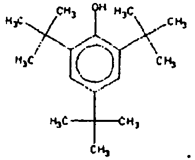

Phosphites and phosphonites are known as processing stabilizer type antioxidants for plastics, including aromatic phosphites and phosphonites. Phosphites and phosphonites useful as one or more of the highly concentrated additives in the present invention have a melting or softening point in the range of about 80°C to about 210°C, more preferably in the range of about 100°C to about 200°C. Phosphites and phosphonites particularly useful in the present invention include, but are not limited to:
(a) AO-168, or tris-(2,4-di-t-butylphenyl) phosphite, having the structure below: 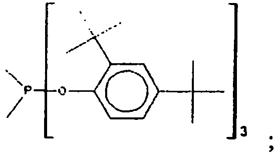
(b) AO-626, or bis (2,4-di-t-butylphenyl) penta-erythritol diphosphite, having the structure below: 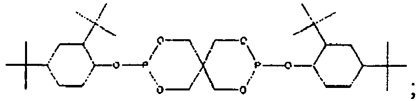
(c) AO-641, or 2,4,6 tri-t-butylphenyl 2 butyl 2 ethyl 1,3 propane diol phosphite, having the structure below: 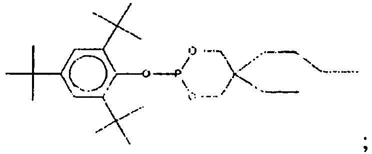 or
(d) AO-PEPQ, or Tetrakis(2,4-di-tert-butylphenyl)[1,1-biphenyl]-4,4'-diyl-bis-phosphonite, having the structure shown below:

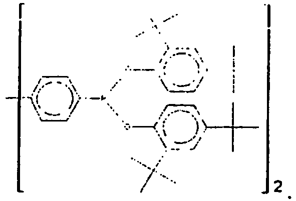

Acid neutralizers that may be used include metallic stearate, such as calcium or zinc stearate and others, magnesium aluminum hydroxy-carbonate hydrate, also known as synthetic hydrotalcite, zinc oxide, and mixtures thereof.

Hindered amines useful in the present invention are principally known as hindered amine light stabilizers ("HALS"). They contain one or more groups of the chemical formula shown below:

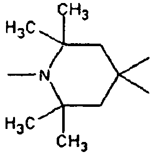

These compounds can be of low or high molecular weight and can be monomeric or oligomeric in nature. The HALS additives useful as the highly concentrated additive in the present invention should have a melting point in the range of about 50°C to about 210°C. More preferably, HALS useful in the present invention have a melting point in the range of about 100°C to about 200°C. HALS useful as the highly concentrated additive in the present invention include, but are not limited to:
(a) HALS-119, having the general structure shown below: 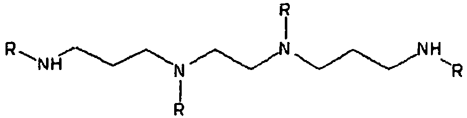 where R is 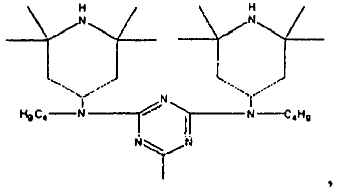 also known as 1,3,5-Triazine-2,4,6-triamine,N,N"'-[1,2-ethane-diyl-bis[[[4,6-bis-[butyl (1,2,2,6,6-pentamethyl-4-piperidinyl)amino]-1,3,5-triazine-2-yl] imino]-3,1-propanediyl] ] bis [N',N"-dibutyl-N',N"-bis(1,2,2,6,6-pentamethyl-4-piperidinyl)-};
(b) HALS-944, having the structure shown below: 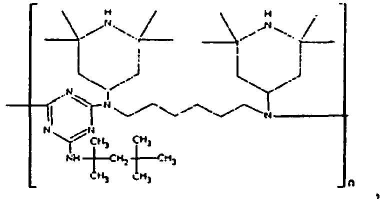 where n is 1 or greater,
   also known as Poly[[6-[(1,1,3,3-tetramethylbutyl)amino]-1,3,5-triazine-2,4-diyl][(2,2,6,6-tetramethyl-4-piperidinyl)imino]-1,6-hexanediyl[(2,2,6,6-tetramethyl-4-piperidinyl)imino]]} ;
(c) HALS 20, having the structure shown below: 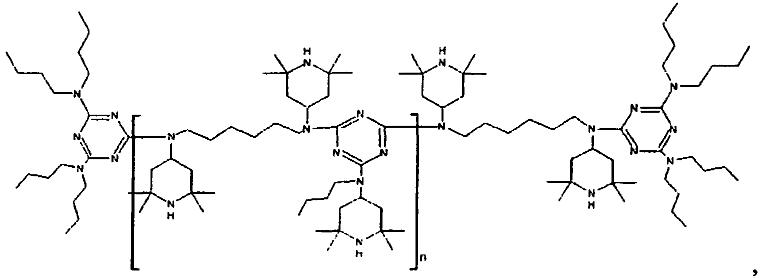 where n is 0 or any positive even integer (e.g. 0, 2, 4, 6, 8...), also known as 1,6-Hexanediamine, N, N'-bis(2,2,6,6-tetramethyl-4-piperidinyl)-polymer with 2,4,6-trichloro-1,3,5-triazine, reaction products with N-butyl-1-butanamine an N-butyl-2,2,6,6-tetramethyl-4-piperidinamine;
(d) HALS-3346, having the structure shown below: 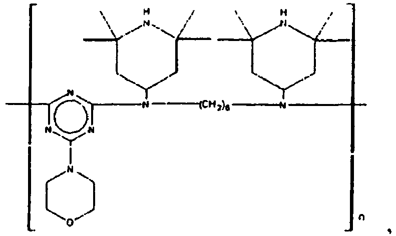 where n is 1 or greater,
   also known as Poly [(6-morpholino-s-triazine-2,4-diyl)[2,2,6,6-tetramethyl-4-piperidyl) imino]- hexamethylene [(2,2,6,6-tetramethyl-4-piperidyl) imino]];
(e) HALS-3529, having the structure shown below: 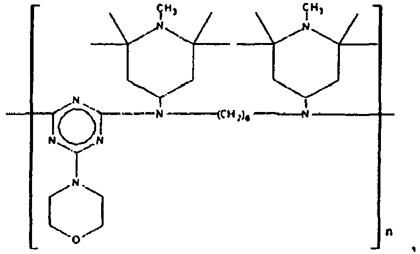 where n is I or greater,
   also known as 1,6-hexanediamine, N,N'-bis(2,2,6,6-tetramethyl-4-piperidinyl)-, polymers with morpholine-2,4,6-trichloro-1,3,5-triazine;
(f) HALS-144, having the structure shown below: 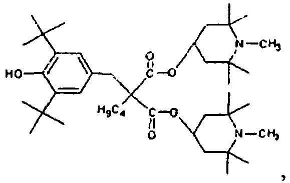 also known as bis(1,22,6,6-pentamethyl-4-piperidinyl)-2-butyl-2-(4-hydroxy-3,5-di-tert-utylbenzyl) propanedioate;
(g) HALS-4050, having the structure shown below: 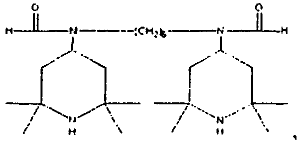 also known as {N,N'-bisformyl-N,N'-bis-(2,2,6,6-tetramethyl-4-piperidinyl)-hexamethylendiamine}; or
(h) HALS-5050, having the structure shown below: 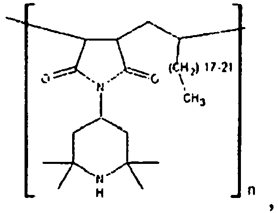 where n is 1 or greater.

The HALS additives can be either monomeric or an oligomeric sterically hindered amines.

Triazines useful in the present invention as UV light absorbers contain one or more groups of the formula shown below:

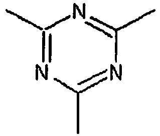

Triazines useful as the highly concentrated additive in the present invention should have a melting point in the range of about 80°C to about 210°C. More preferably, triazines useful in the present invention have a melting point in the range of about 100°C to about 200°C. Triazines useful as the highly concentrated additive in the present invention include, but are not limited to:
LS-1164, or {2-(4,6-bis-(2,4-dimethylphenyl)-1,3,5-triazin-2-yl)-5-(octyloxy)-phenol}, having the structure shown below:

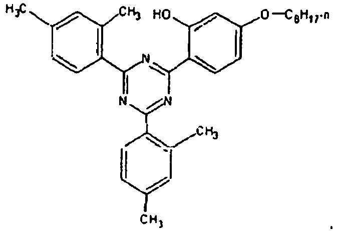

Benzophenones useful in the present invention are principally known as UV light absorbers. They contain one or more groups of the formula shown below:

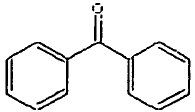

Benzophenones useful as the highly concentrated additive in the present invention should have a melting point in the range of about 80° to about 210°C. More preferably, benzophenones useful in the present invention have a melting point in the range of about 100°C to about 200°C.

Benzotriazoles useful in the present invention are also principally known as light absorbers. They contain one or more groups of the formula shown below:

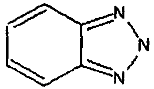

Benzotriazoles useful as the highly concentrated additive in the present invention should have a melting point in the range of about 80°C to about 210°C. More preferably, benzotriazoles useful in the present invention have a melting point in the range of 100°C to about 200°C. Benzotriazoles useful as the highly concentrated additive in the present invention include, but are not limited to:
(a) LS-234, or 2-(2H-benzotriazol-2-yl)-4,6-bis(1-methyl-1-phenylethyl)phenol, having the structure shown below: 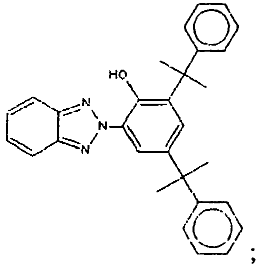
(b) LS-326, or 2-(3'-tert-butyl-2'-hydroxy-5'-methylphenyl)-5-chlorobenzotriazole, having the structure shown below: 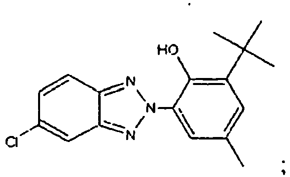
(c) LS-327, or 2-(3',5'-di-tert-butyl-2'-hydroxyphenyl)-5-chlorobenzotriazole, having the structure shown below: 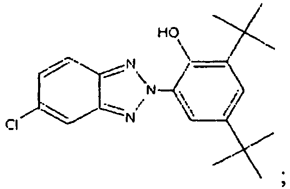
(d) LS-328, or 2-(2H-benzotriazol-2-yl)-4,6-ditertpentylphenol, having the structure shown below: 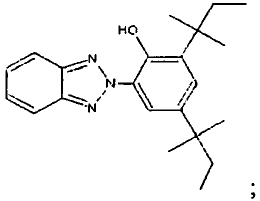
(e) LS-329, or 2-(2H-benzotriazol-2-yl)-4-(1,1,3,3-tetramethylbutyl)phenol, having the structure shown below: 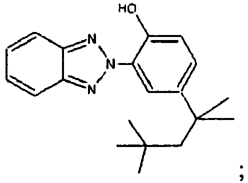 or
(f) LS-3033, or 2-(2H-benzotriazole-2-yl)-4-methylphenyl, having the structure shown below:

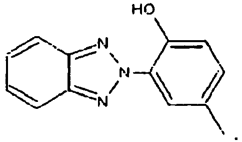

Hydroxybenzoates useful as UV light absorbers in the present invention should have a melting point in the range of about 80°C to about 210°C. More preferably, hydroxybenzoates useful in the present invention have a melting point in the range of about 100°C to about 200°C. Hydroxybenzoates useful as the highly concentrated additive in the present invention include, but are not limited to:
LS-340, or 2,4-di-tert-butylphenyl 3,5-di-t-butyl-4-hydroxybenzoate, having the structure shown below:

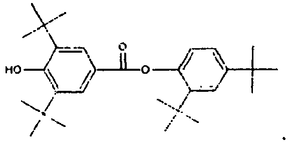

Antistatic agents that may be used as part of the base additive blend include glyceryl monostearate ("GMS"), ethoxylated amines, and others, including mixtures thereof.

One example of a metal deactivator is 2,3-bis (3-(3,5-di-teriary-butyl-4-hydroxyphenyl) propionyl) propionohydrazide; others could be used as well, including mixtures thereof.

Slip agents can include, for example, erucamide, oleamide, behenamide, erucyl erucamide, and others, including mixtures thereof.

Antiblock agents that can be included in the additive blend include synthetic or natural silica, sodium calcium alumino-silicate, and others, including mixtures thereof.

Nucleating agents that can be included in the additive blend include sodium benzoate; sorbitol acetals such as 1,3:2,4-bis(3,4-dimethylbenzylidene) sorbitol; sodium aromatic phosphates such as sodium 2,2'-methylenebis(4,6-di-tertiary-butylphenyl) phosphate; sodium adipate and others, including mixtures thereof.

The compaction aids that accompany the base additive mixture can include metallic silicates, montmorillonite clay, silica, calcium carbonate, barium sulfate, titanium dioxide, zinc oxide, and mixtures thereof.

Metallic silicates useful as the compaction aid additive in the present invention should have a melting point in above 250°C. More preferably, metal silicates useful in the present invention have a melting point above about 300°C. Metallic silicates useful as the compaction aids in the present invention include, but are not limited to, potassium magnesium alumino-silicates, sodium calcium alumino-silicates, anhydrous aluminum silicates, sodium potassium alumino-silicates, calcium silicates, hydrated magnesium silicates, such as talc, sodium alumino-silicates, synthetic magnesium sodium lithium fluoro-silicate, synthetic magnesium sodium lithium phosphated fluoro-silicate, or mixtures thereof.

The potassium magnesium alumino-silicates can be phlogopite mica, Muscovite mica, or mixtures thereof. If the compaction aid is made up of phlogopite mica or Muscovite mica, these should be present at about 0.5% to about 20% by weight of the additive blend, and preferably 3 to 10% of the additive blend. The phlogopite mica or Muscovite mica can also be present at about 15 to about 20% by weight of the additive blend, such as when the base additive mixture has a melting temperature of less than 90°C, as measured by differential scanning calorimetry or other suitable instrument, or in other circumstances if such a concentration is desirable.

The montomorrillonite clay used as a compaction aid can be modified with a quaternary ammonium salt.

Titanium dioxide used as a compaction aid can be present at about 0.5% to about 20% by weight of the additive blend. The titanium dioxide can also be coated with a metallic stearate, such as calcium stearate, magnesium stearate, or zinc stearate. In addition, the compaction aid can be a mixture of titanium dioxide and mica, either phlogopite or Muscovite. If such a mixture is used, then the titanium dioxide should preferably be present at about 0.5% to about 10% by weight of the additive blend, and the mica should be present at about 0.5% to about 10% of the additive blend.

The compaction aids can also include synthetic amorphous silica, present at about 0.5% to about 20% by weight of the additive blend, preferably 3 to 10% of the additive blend.

The primary polymer additive or additives present at total 100% concentration (including compaction aid) in the Superblend of the present invention can include additives known to those in the art, such as antioxidants, light stabilizers, acid neutralizers, and others not listed. Specific examples of the additives include hindered phenolic antioxidants, phosphites, phosphonites, hindered amines, triazines, benzophenones, benzotriazoles, and metal stearates where the peak melting temperature of the additive is in the range of temperatures typically used to process polyolefins, or about 150°C to about 300 °C. Additionally, the Superblend may contain other additives and metal silicate, silica, or others.

Typically, when preparing a pelletized additive blend (e.g. Ingenia Superblend^{™}), by way of example, an antioxidant blend, the compacting operation is carried out using ambient temperature feed. During processing the temperature of the additive mixture rises typically to about 50 to about 90 °C. Subsequently the temperature of the compacted additive pelletized blend drops to ambient temperature. Poor binding of the additives can lead to the formation of dust from the blend. It has surprisingly been found that the friability of the compacted additive pellet is greatly reduced by the addition of solid high melting metallic silicate, silica and other compaction aids to the base additive mixture.

The current invention also pertains to a solventless compaction process for producing compacted additive blends using high melting or non-melting non-migratory solids as compaction aids. These solids include silicates and do not melt during the compaction process. After the preparation of carrierless (i.e. no polymer present) additive blends, the compacted mixture of polymer additives (or additive blend) is fed to an extruder or other mixing device and forced through a die and cut to produce plastic pellets. During mixing, one or more of the additives is melted, such that the blend temperature is above the melting temperature of some of the additives. In some cases, none of the additives may melt in the polymer melt matrix during extrusion. The mixing step is carried out typically in a twin-screw or single screw extruder, whereby the dispersion and processing ease of the additives are improved. This leads to an efficient mixing of the additives within the extruder at regions such as between the extruder screw and die wall or in mixing sections containing kneading blocks or other mixing devices.

In the compaction process the additives blend, which may consist of a phenolic and phosphite antioxidant and a metallic silicate type or other compaction aid, is fed to a compaction mill and forced through a die to produce pellets. During compaction the polymer additives are subjected to compression and shear forces which cause may attrition of the particles even as they are being compacted into pellets.

The first step in this compaction process is the feeding of the additives to the pellet mill. The individual additives may be pre-blended and then fed to the afore-mentioned pellet mill or they may be metered independently. Pre-blending may be accomplished by weighing components and tumble mixing them together. Blending may be accomplished by any means known in the art, including, but not limited to hand mixing, tumble blending, ribbon blending, and high-intensity mixing. Metering additives independently can be accomplished volumetrically or by loss-in-weight feeder by any means known in the art.

The additives or the additive blend are then fed into a pellet mill. The pellet mill may be of any type known to those in the art. Manufacturers of such types of mills include California Pellet Mill (Crawfordsville, Indiana), described in U.S. Patent No. 4,080,134, Kahl and Munch (Germany), and Bliss Industries (Ponca City, Oklahoma). The pellet mill forms a pellet by forcing the additive blend through (a) a rotating cylindrical die and a fixed roller, e.g. CPM and Munch mills or (b) a fixed die and rotating roller, e.g. Kahl mill. Shear and compression between the roller and die and also between the material and die can cause attrition of the granular or powder additives as they are being compacted into pellets. The strand pellets formed are considerably larger than the particles of the granular or powder additives. The compaction process of this invention involves blending of the additives and the compaction aids, providing reduced friability, that is, increased resistance to attrition of the compacted additive blend pellets.

During the compaction of a low-melting (typically less than 90°C, measured by differential scanning calorimetry or DSC) additive mixture a problem can be experienced during processing, namely formation of melt inside the mill. In such an undesirable situation the mill has to be stopped or shut down, followed by removal of the additives and subsequent cleanup of the machine. The melt phenomenon can be mitigated by proper selection of dies. Generally low L/D (length-to diameter ratio) dies reduce the tendency of melt formation. When silicate or other binding aids of this invention are added at 10 to 25% wt concentration to the base low-melting additive mixture, the melt phenomenon in the compaction chamber is significantly reduced, thereby increasing the operating efficiency of the process. Preferably 15 to 20% wt of the silicate or other compaction aid of this invention is added to the base additive mixture to minimize the melt phenomenon in the mill.

The current invention also pertains to a method for preparing a polymer resin. The first step is to heat the base polymer to produce a molten polymer stream. Next, the additive blend described above is added to the molten polymer stream to produce an additivized polymer stream. Finally, the treated polymer stream is allowed to cool and harden, to produce a polymer resin. In preferred embodiments, the additive stream can be added without a polymer carrier.

### EXAMPLES

The compaction of the additives in the examples described below was performed in a laboratory model California Pellet Mill ("CPM") pelletizer. In this process, the mixture of additives is forced through a rotating die, thereby producing compression and compacting of the additives into a rod-like strands, which are cut into smaller pieces of pellet.

Friability was measured by testing the particle size distribution of the afore-mentioned pellets by mechanical sieve shaking of pellets as produced and sieving of the pellets when steel ball bearings were placed in the screens. In the latter case the impact of the balls generated attrition effect, thus causing reduction of particle size of the pellets and formation of fine powder. High attrition of pellets indicates high friability, which is an undesirable effect. A combination of large and small ball bearings were used for impact during the sieve shaking. The sieve stacks range from 0.265 to 200 mesh opening. The large balls can have diameter of 0.25 to 1 inch. The small balls can have diameter of 1/16 to ½ inch. The sieves were shaken typically for 5 to 15 minutes.

For attrition or friability testing of compacted pellets, the sieve test result of attrited pellet was compared to that of initial or un-attrited pellets. The sieves were stacked with the coarsest mesh size (biggest opening) at the top, with the consecutive finer screen beneath it and the pan at the bottom of the stack. The sequence of sieves was 0.265 mesh (top), then 4, 8, 14, 18, 120, 200 mesh, and pan (bottom). A 100 gm pellet sample was placed on the top of the stacked sieves. The sieve stack was shaken for 5 minutes on a mechanical sieve shaker. For the attrition test, ball bearings were added to the sieves as follows: seven 5/8 inch diameter and twenty ¼ inch diameter bearings on 4 mesh sieve; three 5/8 inch and ten ¼ inch bearings on 8 mesh sieve; ten ¼ inch bearings on 14 mesh sieve; and five ¼ inch bearings on 18 mesh sieve. After shaking with these bearings, the earlier procedure was followed.

Two parameters were used to characterize the effect of attrition of the pellets. They relate to measures of the attrition performance of the pellets. From a typical particle size distribution curve, the 50^{th} percentile median particle size of the initial pellets is reported as PS₀, and that of the pellets after attrition is reported as PS₁. The Attrition Index (or friability index) is defined as (PS₁/PS₀) X 100, expressed as a percentage. If there is no attrition at all, PS₁ = PS₀ so that the attrition index is 100%. As attrition increases, average particle size of the attrited pellets is reduced and PS₁ decreases. That reduces the attrition index. A higher value of the attrition index indicates better attrition performance, that is, less reduction of particle size after milling with ball bearings.

Another measure of friability performance is reported here, namely the percentage change of particle mass retained on 14 mesh screen, after attrition test. If this number is small (meaning less change), less attrition or better attrition performance of the pellet is indicated. This parameter will be referred to as 14 mesh attrition or friability. Note that during the sieve shaking test, the 14 mesh screen was placed in the middle of the stack of seven screens.

The additives used in the experiments of the examples are described here. Evernox® 10 (Everspring Chemical) is a hindered phenolic primary antioxidant ("AO") which is used for processing and long term heat aging stability. It has a melting temperature range of about 110°C to about 125°C. Erucamide is a fatty amide used as slip agent in polymer film. It has a melting temperature of about 80-85°C. GMS-52 is a glyceryl monostearate ("GMS") with 52% alpha-mono stearate content. It is used as an antistatic agent, lubricant and mold release agent in polymers. It has a melting temperature of about 50°C. Sodium benzoate is a heterogeneous nucleating agent used for enhancing the crystallization rate and reducing the molding cycle time of polymers like polypropylene ("PP"). Sodium benzoate is a high melting (>300°C) additive and remains unmelted in the conventional polymer extrusion process.

The following solid (i.e., non-melting under compaction conditions) compaction aids were used in the experiments described in the examples below, each additive being used at 5.0 wt% level. Wollastonite Nyad® 5000 was obtained from Nyco Minerals (Calgary, Alberta, Canada). This is calcium silicate of 2.2 micron median particle size.

Minbloc® HC-400 (from Unimin, New Canaan, Connecticut) is sodium-potassium alumino-silicate of 2.8 micron median particle size.

Kaopolite® SF (from Imerys, Paris, France) is anhydrous A1 silicate of 0.7 micron median particle size. Kaopolite® 1147 (from Imerys) is anhydrous A1 silicate of 1.6 micron median particle size.

Sylobloc® 45 (from Grace Davison, Columbia, Maryland) is a synthetic amorphous silica of about 3-4 micron mean particle size. It has a citric acid coating.

Silton® JC30 (from Mizusawa Chemical, Tokyo, Japan) is sodium calcium alumino-silicate, of about 3 micron mean particle size. Silton® JC50 (Mizusawa Chemical) is sodium calcium alumino-silicate, of about 5 micron mean particle size.

Suzorite mica 400-HK (from Kings Mountain Minerals / Zemex Industrial Minerals, Atlanta, Georgia) is potassium-magnesium alumino-silicate of 15 micron mean particle size. It is a phlogopite type mica.

Mica C-4000 is a Muscovite mica from Zemex Industrial Minerals (Kings Mountain Mining, Kings Mountain, North Carolina) of mean particle size 17 micron, aspect ratio 6, and BET surface area of 6.3 square meter per gram.

Zinc oxide AZO77HSA (from U.S. Zinc, Houston, Texas) has 0.1 micron mean particle size, produced by the French process.

Barium sulfate used was designated Bartex® 65, from Hitox (Corpus Christi, Texas).

Hydrotalcite DHT-4V (from Kisuma / Kyowa Chemical, Tokyo, Japan) is magnesium aluminum hydroxy carbonate hydrate, and has about 0.4 mean particle size. It has a stearate coating which is vegetable derived.

Three talc grades were used, all from Rio Tinto Minerals (Greenwood Village Colorado, formerly Luzenac, Melbourne, Australia). Cimpact® 610 is powder with 3.2 micron median particle size. Cimpact® 550C is a compacted talc, from 3.6 micron median primary particle size. Cimpact® 710C is a compacted talc, with 1.8 micron median particle size.

Sipernat® 44MS is sodium aluminum silicate (from Evonik, formerly Degussa, Essen, Germany) of mean particle size about 3.5 micron.

Cloisite® 15A and Cloisite® Na+ are nano-clays from Southern Clay Products (Gonzales, Texas). For both clays 90% by volume passes through 13 micron screen. Cloisite® 15A is alkyl quaternary ammonium bentonite. It is a natural montmorillonite modified with a quaternary ammonium salt. The organic modifier is dimethyl, di-hydrogenated tallow quaternary ammonium with chloride anion. Cloisite® Na+ is unmodified Montmorillonite or hydrated aluminum silicate.

Laponite® B (Southern Clay Products) is synthetic layered magnesium sodium lithium fluoro-silicate, typically with 55% SiO₂, 27% MgO, 1.4% Li₂O,3.8% Na₂O and 5.6% F. Laponite S is synthetic layered magnesium sodium lithium phosphated fluoro-silicate, typically with 51% SiO₂, 25% MgO, 6% Na₂O, 1.3% Li₂O, 3.3% P₂O₅ and 5.0% F.

For a comparative example, a neat or 100% additive blend was prepared using the following steps. The additive blend E0 (see Table 1 below for composition) was prepared by weighing out the individual additives, then mixing them in a Prodex Henschel (Kassel, Germany) intensive mixer. Evernox® 10 (Everspring Chemical) was dry blended with erucamide (Crodamide ER), GMS-52 (Pationic® 1052 from Caravan, Lenexa, Kansas), and sodium benzoate 20M (Tulstar, Tulsa, Oklahoma). This additive blend (with no polymer) was then fed to a laboratory model California Pellet Mill and processed into pellets. The CPM compactor was run with a I inch long and 3/16 inch diameter die, that is, length to diameter ratio (L/D) of 5.3, speed of 521 revolutions per minute ("RPM"), feed rate of about 316 lb/hour, and motor load of about 60%. No external heating or cooling was used. The pelletized strands were cut by a knife-blade, then collected in a bucket. The mixture of pellets and powder were sieved through 6 mesh screen, and the powder was recycled. Good strands were formed under stable compaction conditions. The resulting pellets showed some powder or dust on the surface of the pellets. The peak melting temperature (by DSC) of the pellet at 10°C/minute heating rate was 63.0°C.

Unprocessed bulk density of the initial powder mixture was 53.2 gm/100 cc. Table 1 below shows the results for the pellets produced. The attrition index was 11.2% and the 14 mesh attrition of the pellets was measured as 89%, indicating high level of attrition of the pellet in this comparative example.

In Examples I through 23 below, the same base formulation of additive mixture was used as described in the comparative example above, but additionally 5% by weight of compaction aid was added. The results are shown in the tables below. The weighed compaction aid was added to the mixture of four additives processed earlier in the Henschel intensive mixer, and the resulting blend of five additives was hand tumbled in a plastic bag. The final blend was then compacted in the CPM pelleter following the same operating procedure as in the comparative example above. For Examples 1-23, the unprocessed additive powder mixture had a bulk density of 53 gm/100 cc. Table I below shows the formulation and pellet properties of compacted additive mixtures described in Examples 1-7 below, including attrition testing.

**Table 1**

| **Formulation (wt%)** | Comparative example (E0) | Ex. 1 | Ex. 2 | Ex. 3 | Ex. 4 | Ex. 5 | Ex. 6 | Ex. 7 |
|---|---|---|---|---|---|---|---|---|
| Evernox 10 | 9.3 | 8.8 | 8.8 | 8.8 | 8.8 | 8.8 | 8.8 | 8.8 |
| Erucamide | 40.2 | 38.2 | 38.2 | 38.2 | 38.2 | 38.2 | 38.2 | 38.2 |
| GMS (Pationic 1052) | 46.55 | 44.2 | 44.2 | 44.2 | 44.2 | 44.2 | 44.2 | 44.2 |
| Na benzoate (20M) | 3.95 | 3.8 | 3.8 | 3.8 | 3.8 | 3.8 | 3.8 | 3.8 |
| Wollastonite Nyad 5000 | | 5 | | | | | | |
| Minbloc HC400 | | | 5 | | | | | |
| Kaopolite SF | | | | 5 | | | | |
| Kaopolite 1147 | | | | | 5 | | | |
| Silica Sylobloc 45 | | | | | | 5 | | |
| Silton JC30 | | | | | | | 5 | |
| Mica phlogopite 400HK | | | | | | | | 5 |

| **Pellet properties** | | | | | | | | |
|---|---|---|---|---|---|---|---|---|
| Bulk density(g/100cc) | 50.5 | 49.9 | 51.3 | 51.3 | 51.3 | 51.3 | 51.3 | 51.3 |
| Mean PS (micron), initial, PS₀, 50^{th} percentile | 5600 | 5400 | 5500 | 5600 | 5500 | 5550 | 5625 | 5800 |
| Mean PS (micron), PS₁, after attrition, 50th percentile | 625 | 800 | 900 | 750 | 1050 | 900 | 1050 | 1200 |
| Attrition index, (PS₁/PS₀) X100, % | 11.2 | 14.8 | 16.4 | 13.4 | 19.1 | 16.2 | 18.7 | 20.7 |
| 14 mesh attrition (% change) | 89 | 74 | 68 | 80 | 60 | 66 | 55 | 53 |

### EXAMPLE 1

In Example 1, 5 wt% wollastonite Nyad® 5000 powder was used. The attrition index was 14.8% and the 14 mesh attrition was 74%, indicating improvement compared to the control sample (comparative example). Lower 14 mesh attrition numbers are more desirable. It indicates less change of the attrited pellets, compared to the unattrited pellets. The resulting pellets were observed to have a smooth outer surface.

### EXAMPLE 2

The same base formulation of powder additive mixture was used as in Example 1, but 5% by weight Minbloc® HC400 powder was used as compaction aid. This mixture of five additives was dry tumbled and then processed in the CPM machine, using the same procedure as in Example 1. The attrition index was 16.4% and the 14 mesh attrition was 68%. The resulting pellets were observed to have a smooth outer surface.

### EXAMPLE 3

The same base formulation of powder additive mixture was used as in Example 1, but 5% by weight Kaopolite® SF powder was used. This mixture of five additives was dry tumbled and then compacted in the CPM machine using the same procedure as in Example 1. The attrition index was 13.4% and the 14 mesh attrition was 80%. The resulting pellets were observed to have a smooth outer surface.

### EXAMPLE 4

The same base formulation of powder additive mixture was used as in Example 1, but 5% by weight Kaopolite® 1147 powder was used as compaction aid. This mixture of five additives was dry tumbled and then compacted in the CPM pelleter using the same procedure as in Example 1. The resulting pellets were observed to have a smooth outer surface. The attrition index was 19.1% and the 14 mesh attrition of the pellet was measured as 60%.

### EXAMPLE 5

The same base formulation of powder additive mixture was used as in Example 1, but 5% by weight Sylobloc® 45 powder was used as compaction aid. This mixture of five additives was dry tumbled and then compacted in the CPM machine using the same procedure as in Example 1. The resulting pellets were observed to have a smooth outer surface. The attrition index was 16.2% and the 14 mesh attrition of the pellet was measured as 66%.

### EXAMPLE 6

The same base formulation of powder additive mixture was used as in the comparative example, but additionally 5% by weight Silton® JC30 powder was used. This mixture of five additives was dry tumbled and then compacted in the CPM machine using the same procedure as in Example 1. The resulting pellets were observed to have a smooth outer surface. The attrition index was 18.7% and 14 mesh attrition of the pellet was measured as 55%. The pelletized strand had smooth surface appearance.

### EXAMPLE 7

The same base formulation of powder additive mixture was used as in the comparative example, but additionally 5% by weight phlogopite mica Suzorite 400-HK powder was used. This mixture of five additives was dry tumbled and then compacted in the CPM pelleter using the same procedure as in the comparative example. The resulting pellets were observed to have a smooth outer surface. The attrition index of the pellet was 20.7% and 14 mesh attrition was measured as 53%.

Table 2 below shows the formulation and pellet properties of compacted additive mixtures, including attrition testing results for Examples 8-14.

**Table 2**

| **Formulation (wt%)** | Ex. 8 | Ex. 9 | Ex. 10 | Ex. 11 | Ex. 12 | Ex. 13 | Ex. 14 |
|---|---|---|---|---|---|---|---|
| Evernox 10 | 8.8 | 8.8 | 8.8 | 8.8 | 8.8 | 8.8 | 8.8 |
| Erucamide | 38.2 | 38.2 | 38.2 | 38.2 | 38.2 | 38.2 | 38.2 |
| GMS (Pationic 1052) | 44.2 | 44.2 | 44.2 | 44.2 | 44.2 | 44.2 | 44.2 |
| Na benzoate 20M | 3.8 | 3.8 | 3.8 | 3.8 | 3.8 | 3.8 | 3.8 |
| Mica phlogopite 400HK - 50PA | 5 | | | | | | |
| Mica Muscovite C-4000 | | 5 | | | | | |
| Silton JC50 | | | 5 | | | | |
| Zn oxide | | | | 5 | | | |
| Ba sulfate | | | | | 5 | | |
| Calcium carbonate | | | | | | 5 | |
| Titanium dioxide | | | | | | | 5 |

| **Pellet properties** | | | | | | | |
|---|---|---|---|---|---|---|---|
| Bulk density(g/100cc) | 51.8 | 54.7 | 51.8 | 51.7 | 52.6 | 51.4 | 54.7 |
| Mean PS (micron), initial 50th percentile, PS₀ | 6000 | 6000 | 6000 | 6000 | 5900 | 5800 | 5800 |
| Mean PS (micron), after attrition, 50th percentile, PS₁ | 1200 | 1210 | 920 | 910 | 910 | 905 | 1110 |
| Attrition index, (PS₁/PS₀) X100, % | 20 | 20.2 | 15.3 | 15.2 | 15.4 | 15.6 | 19.1 |
| 14 mesh attrition (% change) | 56 | 54 | 66 | 66 | 67 | 68 | 60 |

### EXAMPLE 8

The same base formulation of powder additive mixture was used as in the comparative example, but additionally 5% by weight phlogopite mica Suzorite 400-HK powder was used (a different sample). This mixture of five additives was dry tumbled and then compacted in the CPM pelleter using the same procedure as in the comparative example. The resulting pellets were observed to have a smooth outer surface. The attrition index was measured as 20.0%, compared to 10.7% for the comparative example. The 14 mesh attrition of the pellet was measured as 53%. Both parameters indicate improvement over the comparative example (or control, that is without compaction aid).

### EXAMPLE 9

The same base formulation of powder additive mixture was used as in the comparative example, but additionally 5% by weight Muscovite mica C-4000 powder was used. This mixture of five additives was dry tumbled and then compacted in the CPM pelleter using the same procedure as in the comparative example. The resulting pellets were observed to have a smooth outer surface. The attrition index was measured as 20.2%. The 14 mesh attrition of the pellets was 54%. Both parameters indicate improvement in attrition performance or resistance to friability, over the comparative example (control).

### EXAMPLES 10-14

The formulations and test results are shown in Table 2 above. Silton® JC50, zinc oxide, barium sulfate, calcium carbonate and titanium dioxide improved the attrition performance over the comparative example. DHT-4V and Cloisite® Na+ showed only marginal or only slight improvement in attrition performance over the comparative example.

Table 3 below shows the formulation and pellet properties of compacted pellets, including particle size (PS) before and after attrition.

**Table 3**

| **Formulation (wt%)** | Ex. 15 | Ex. 16 | Ex. 17 | Ex. 18 | Ex. 19 | Ex. 20 | Ex. 21 | Ex. 22 | Ex. 23 |
|---|---|---|---|---|---|---|---|---|---|
| Evernox 10 | 8.8 | 8.8 | 8.8 | 8.8 | 8.8 | 8.8 | 8.8 | 8.8 | 8.8 |
| Erucamide | 38.2 | 38.2 | 38.2 | 38.2 | 38.2 | 38.2 | 38.2 | 38.2 | 38.2 |
| GMS (Pationic 1052) | 44.2 | 44.2 | 44.2 | 44.2 | 44.2 | 44.2 | 44.2 | 44.2 | 44.2 |
| Na benzoate 20M | 3.8 | 3.8 | 3.8 | 3.8 | 3.8 | 3.8 | 3.8 | 3.8 | 3.8 |
| DHT-4V | 5 | | | | | | | | |
| Talc - Cimpact 610 | | 5 | | | | | | | |
| Talc - Cimpact 550C | | | 5 | | | | | | |
| Talc - Cimpact 710C | | | | 5 | | | | | |
| Sipernat 44MS | | | | | 5 | | | | |
| Cloisite 15A | | | | | | 5 | | | |
| Cloisite Na+ | | | | | | | 5 | | |
| Laponiie B | | | | | | | | 5 | |
| laponite S | | | | | | | | | 5 |

| **Pellet properties** | | | | | | | | | |
|---|---|---|---|---|---|---|---|---|---|
| Bulk density(g/100cc) | 50.5 | 51.5 | 51.7 | 52.6 | 50.7 | 52.3 | 49.3 | 53.9 | 54.4 |
| Mean PS (micron), | 6000 | 5900 | 5900 | 5760 | 5750 | 5800 | 5740 | 5650 | 5600 |
| initial, PS0, 50th percentile | | | | | | | | | |
| Mean PS (micron), PS1, after attrition, 50th percentile | 760 | 900 | 895 | 880 | 1000 | 905 | 800 | 1000 | 950 |
| Attrition index, (PS1/PS0) X100 | 12.7 | 15.3 | 15.2 | 15.3 | 17.4 | 15.6 | 13.9 | 17.7 | 17 |
| 14 mesh attrition (% change) | 76 | 72 | 74 | 69 | 70 | 66 | 71 | 58 | 62 |

### EXAMPLES 15-23

The formulations and test results are shown in Table 3 above. Talc (three grades), Sipernat® 44 and Cloisite® 15A improved the attrition performance of compacted pellets over the comparative example.

### EXAMPLES 15-23

In the following example and the comparative example (E00), a different die, namely 1.25 inch long and 3/16 inch diameter (L/D ratio of 6.7) was used, and other procedural and processing conditions were similar to those of Example 2. In comparative example E00 below the additive formulation was the same as in the comparative example in Table I above. In Example 24 the same additive formulation was used as in Example 2, namely with 5% by weight Minbloc® HC400 compaction aid powder. This mixture of five additives was dry tumbled and then compacted in the CPM machine, using the same procedure as in Example 2. For the comparative example E00 the attrition index was 38.8% and the 14 mesh attrition was 38.9%. For Example 24, the attrition index was 72.4% and the 14 mesh attrition was 20.2%. Both parameters for the Example 24 indicate improvement over the comparative example.

Table 4 below shows the formulation and attrition properties of compacted pellets, including particle size (PS) distribution before and after attrition, using a different die.

| **Formulation (wt%)** | Comparative example (E00) | Example 24 |
|---|---|---|
| Evernox 10 | 9.3 | 8.8 |
| Erucamide | 40.2 | 38.2 |
| GMS (Pationic 1052) | 46.55 | 44.2 |
| Na benzoate (20M) | 3.95 | 3.8 |
| Minbloc HC400 | | 5 |
| **Pellet properties** | | |
| Median PS (micron), initial, PS₀, 50th percentile | 5800 | 5800 |
| Median PS (micron), PS₁, after attrition, 50th percentile | 2250 | 4200 |
| Attrition index, (PS1/PS0) X100, % | 38.8 | 72.4 |
| 14 mesh attrition (% change) | 38.9 | 20.2 |

Attrition index can be further increased (i.e. improved) by optimizing the concentration of silicate or other compaction aid in the total additive mix; by varying the type and ratio of the base additive mixture; by the use of different die etc. Other minerals including silicate can also increase the attrition index. Using such techniques, attrition index of 80-90% or higher can be potentially achieved.

### REFERENCES CITED

U.S. Patent No. 4,080,134
U.S. Patent No. 5,240,642
U.S. Patent No. 5,597,857
U.S. Patent No. 5,844,042
U.S. Patent No. 5,846,656
U.S. Patent No. 6,033,600
U.S. Patent No. 6,515,052
U.S. Patent No. 6,596,198
U.S. Patent No. 6,740,694
U.S. Patent No. 6,800,228

## Claims

1. A compacted pelletized additive blend for reducing friability of a base polymer, comprising:
(a) a base additive mixture; and
(b) one or more compaction aids selected from the group consisting of metallic silicates, montmorillonite nano-clay, silica, calcium carbonate, barium sulphate, titanium dioxide, zinc oxide, and mixtures thereof, wherein the one or more compaction aids do not melt into liquid in the environment of a compaction mill, and wherein the compacted pelletized additive blend contains no polymer carrier.

2. The additive blend of claim 1, wherein the base additive mixture comprises one or more phenolic antioxidants, processing stabilizers, acid neutralizers, UV light stabilizers, UV light absorbers, antistatic agent, metal deactivators, slip agents, antiblock agents, nucleating agents, lubricants, mold release agents, flame retardants, or mixtures thereof.

3. The additive blend of claim 2, wherein the base additive mixture comprises one or more phenolic antioxidants, and wherein the phenolic antioxidants are hindered phenolic primary antioxidants.

4. The additive blend of claim 2, wherein the base additive mixture comprises one or more processing stabilizers, and wherein the processing stabilizers are phosphites, phosphonites, or mixtures thereof.

5. The additive blend of claim 2, wherein the base additive mixture comprises one or more acid neutralizers, and wherein the acid neutralizers are metallic stearates, magnesium aluminium hydroxyl-carbonate hydrates, zinc oxide, or mixtures thereof.

6. The additive blend of claim 2, wherein the base additive mixture comprises one or more UV light stabilizers, and wherein the UV light stabilizers are hindered amine light stabilizers.

7. The additive blend of claim 2, wherein the base additive mixture comprises one or more UV light absorbers, and wherein the UV light absorbers are triazines, benzophenones, benzotriazoles, hydroxybenzoates, or mixtures thereof.

8. The additive blend of claim 2, wherein the base additive mixture comprises one or more antistatic agents, and wherein the antistatic agents are glyceryl monostearate ("GMS"), ethoxylated amines, or mixtures thereof.

9. The additive blend of claim 2, wherein the base additive mixture comprises one or more metal deactivators, and wherein the metal deactivators are propionohydrazides.

10. The additive blend of claim 2, wherein the base additive mixture comprises one or more slip agents, and wherein the slip agents are erucamide, oleamide, behenamide, erucyl erucamide, or mixtures thereof.

11. The additive blend of claim 2, wherein the base additive mixture comprises one or more antiblock agents, and wherein the antiblock agents are synthetic silica, natural silica, sodium calcium alumino-silicate, or mixtures thereof.

12. The additive blend of claim 2, wherein the base additive mixture comprises one or more nucleating agents, and wherein the nucleating agents are sodium benzoate, sorbitol acetals, sodium aromatic phosphates, sodium adipate, or mixtures thereof.

13. The additive blend of claim 1, wherein the one or more compaction aids are metallic silicates, and wherein the metallic silicates are potassium magnesium alumino-silicates, sodium calcium alumino-silicates, anhydrous aluminium silicates, sodium potassium alumino-silicates, calcium silicates, hydrated magnesium silicates, sodium alumino-silicates, synthetic magnesium sodium lithium fluoro-silicate, synthetic magnesium sodium lithium phosphated fluoro-silicate, or mixtures thereof.

14. The additive blend of claim 13, wherein the metallic silicates are potassium magnesium alumino-silicates, and wherein the potassium magnesium alumino-silicates are phlogopite mica, Muscovite mica, or mixtures thereof.

15. The additive blend of claim 13, wherein the metallic silicates are phlogopite mica or Muscovite mica and wherein the phlogopite mica or Muscovite mica are about 15 to about 20% by weight of the additive blend.

16. The additive blend of claim 1, wherein the one or more compaction aids are selected from
(a) montmorillonite clay, and wherein the montmorillonite clay is modified with a quaternary ammonium salt,
(b) titanium dioxide, and wherein the titanium dioxide is coated with a metallic stearate,
(c) potassium magnesium alumino-silicates and titanium dioxide, and wherein the potassium magnesium alumino-silicates are phlogopite mica, Muscovite mica, or a mixture of, or
(d) synthetic amorphous silicas.

17. The additive blend of claim 1, wherein the base additive mixture has a melting temperature of less than about 90°C.

18. A method for preparing a polymer resin, comprising;
heating a base polymer to produce a molten polymer stream;
adding the compacted pelletized additive blend of claim 1 to the molten polymer stream to produce an additivized polymer stream, wherein the compacted pelletized additive blend lacks a polymer carrier; and allowing the additivized polymer stream to cool and harden, to produce a polymer resin.

19. The method of claim 18, wherein the base polymer is polyethylene, polypropylene, ethylene-propylene copolymers, ethylene-alpha-olefin-copolymers, polybutene-1, polystyrene, ethylene-vinyl acetate copolymers, ethylene vinyl alcohol copolymers, styrene-butadiene copolymers, and mixtures and copolymers thereof.

20. A compacted pelletized additive blend having reduced friability for addition to a base polymer, comprising;
(a) a hindered phenolic primary antioxidant;
(b) erucamide;
(c) glyceryl monostearate;
(d) sodium benzoate; and
(e) one or more compaction aids selected from the group consisting of phlogopite mica, Muscovite mica, titanium dioxide, and mixtures thereof, wherein the compacted pelletized additive blend contains no polymer carrier.

21. The additive blend of claim 20, wherein the one or more compaction aids is phlogopite mica or Muscovite mica, and wherein the phlogopite mica or Muscovite mica is about 0.5 to about 20% by weight of the additive blend.

22. The additive blend of claim 21, wherein the one or more compaction aids is a mixture of titanium dioxide, at about 0.5% to about 10% by weight of the additive blend, and phlogopite mica, Muscovite mica, or mixtures thereof, at about 0.5% to about 10% by weight of the additive blend.

## Patentansprüche

1. Kompaktiertes granuliertes Additivgemisch zum Reduzieren der Bröckligkeit eines Grundpolymers, umfassend:
(a) ein Grundadditivgemisch; und
(b) ein oder mehrere Kompaktierungshilfsmittel, die aus der Gruppe ausgewählt sind, die aus Metallsilicaten, Montmorillonit-Nanoton, Siliciumoxid, Calciumcarbonat, Bariumsulfat, Titandioxid, Zinkoxid und Gemischen davon besteht, wobei das eine oder die mehreren Kompaktierungshilfsmittel in der Umgebung einer Kompaktierpresse nicht zu einer Flüssigkeit schmelzen und wobei das kompaktierte granulierte Additivgemisch keinen Polymerträger enthält.

2. Additivgemisch gemäß Anspruch 1, wobei das Grundadditivgemisch ein oder mehrere phenolische Antioxidantien, Verarbeitungsstabilisatoren, Säureneutralisatoren, UV-Stabilisatoren, UV-Absorber, Antistatikmittel, Metalldeaktivatoren, Gleitmittel, Antiblockmittel, Keimbildner, Schmiermittel, Formentrennmittel, Flammschutzmittel oder Gemische davon umfasst.

3. Additivgemisch gemäß Anspruch 2, wobei das Grundadditivgemisch ein oder mehrere phenolische Antioxidantien umfasst und wobei die phenolischen Antioxidantien primäre Antioxidantien in Form gehinderter Phenole sind.

4. Additivgemisch gemäß Anspruch 2, wobei das Grundadditivgemisch ein oder mehrere Verarbeitungsstabilisatoren umfasst und wobei die Verarbeitungsstabilisatoren Phosphite, Phosphonite oder Gemische davon sind.

5. Additivgemisch gemäß Anspruch 2, wobei das Grundadditivgemisch ein oder mehrere Säureneutralisatoren umfasst und wobei es sich bei den Säureneutralisatoren um Metallstearate, Magnesiumaluminiumhydroxycarbonat-Hydrate, Zinkoxid oder Gemische davon handelt.

6. Additivgemisch gemäß Anspruch 2, wobei das Grundadditivgemisch ein oder mehrere UV-Stabilisatoren umfasst und wobei die UV-Stabilisatoren Lichtstabilisatoren in Form gehinderter Amine sind.

7. Additivgemisch gemäß Anspruch 2, wobei das Grundadditivgemisch ein oder mehrere UV-Absorber umfasst und wobei die UV-Absorber Triazine, Benzophenone, Benzotriazole, Hydroxybenzoate oder Gemische davon sind.

8. Additivgemisch gemäß Anspruch 2, wobei das Grundadditivgemisch ein oder mehrere Antistatikmittel umfasst und wobei es sich bei den Antistatikmitteln um Glycerylmonostearat ("GMS"), ethoxylierte Amine oder Gemische davon handelt.

9. Additivgemisch gemäß Anspruch 2, wobei das Grundadditivgemisch ein oder mehrere Metalldeaktivatoren umfasst und wobei die Metalldeaktivatoren Propionohydrazide sind.

10. Additivgemisch gemäß Anspruch 2, wobei das Grundadditivgemisch ein oder mehrere Gleitmittel umfasst und wobei es sich bei den Gleitmitteln um Erucamid, Oleamid, Behenamid, Erucylerucamid oder Gemische davon handelt.

11. Additivgemisch gemäß Anspruch 2, wobei das Grundadditivgemisch ein oder mehrere Antiblockmittel umfasst und wobei es sich bei den Antiblockmitteln um synthetisches Siliciumoxid, natürliches Siliciumoxid, Natriumcalciumaluminiumsilicat oder Gemische davon handelt.

12. Additivgemisch gemäß Anspruch 2, wobei das Grundadditivgemisch ein oder mehrere Keimbildner umfasst und wobei es sich bei den Keimbildnern um Natriumbenzoat, Sorbitacetale, aromatische Natriumphosphate, Natriumadipat oder Gemische davon handelt.

13. Additivgemisch gemäß Anspruch 1, wobei das eine oder die mehreren Kompaktierungshilfsmittel Metallsilicate sind und wobei es sich bei den Metallsilicaten um Kaliummagnesiumaluminiumsilicate, Natriumcalciumaluminiumsilicate, wasserfreie Aluminiumsilicate, Natriumkaliumaluminiumsilicate, Calciumsilicate, hydratisierte Magnesiumsilicate, Natriumaluminiumsilicate, synthetisches Magnesiumnatriumlithiumfluorosilicat, synthetisches phosphatiertes Magnesiumnatriumlithiumfluorosilicat oder Gemische davon handelt.

14. Additivgemisch gemäß Anspruch 13, wobei die Metallsilicate Kaliummagnesiumaluminiumsilicate sind und wobei es sich bei den Kaliummagnesiumaluminiumsilicaten um Phlogopit-Glimmer, Muscovit-Glimmer oder Gemische davon handelt.

15. Additivgemisch gemäß Anspruch 13, wobei es sich bei den Metallsilicaten um Phlogopit-Glimmer oder Muscovit-Glimmer handelt und wobei der Phlogopit-Glimmer oder Muscovit-Glimmer etwa 15 bis etwa 20 Gew.-% des Additivgemischs ausmacht.

16. Additivgemisch gemäß Anspruch 1, wobei das eine oder die mehreren Kompaktierungshilfsmittel ausgewählt sind aus
(a) Montmorillonit-Ton, wobei der Montmorillonit-Ton mit einem quartären Ammoniumsalz modifiziert ist;
(b) Titandioxid, wobei das Titandioxid mit einem Metallstearat beschichtet ist;
(c) Kaliummagnesiumaluminiumsilicaten und Titandioxid, wobei es sich bei den Kaliummagnesiumaluminiumsilicaten um Phlogopit-Glimmer, Muscovit-Glimmer oder ein Gemisch davon handelt; oder
(d) synthetischen amorphen Siliciumoxiden.

17. Additivgemisch gemäß Anspruch 1, wobei das Grundadditivgemisch eine Schmelztemperatur von weniger als etwa 90 °C aufweist.

18. Verfahren zur Herstellung eines Polymerharzes, umfassend:
Erhitzen eines Grundpolymers unter Bildung eines geschmolzenen Polymerstroms;
Hinzufügen des kompaktierten granulierten Additivgemischs gemäß Anspruch 1 zu dem geschmolzenen Polymerstrom unter Bildung eines mit Additiven versetzten Polymerstroms, wobei das kompaktierte granulierte Additivgemisch keinen Polymerträger enthält; und
Abkühlen- und Härtenlassen des mit Additiven versetzten Polymerstroms unter Bildung eines Polymerharzes.

19. Verfahren gemäß Anspruch 18, wobei es sich bei dem Grundpolymer um Polyethylen, Polypropylen, Ethylen-Propylen-Copolymere, Ethylen-α-Olefin-Copolymere, Polybuten-1, Polystyrol, Ethylen-Vinylacetat-Copolymere, Ethylen-Vinylalkohol-Copolymere, Styrol-Butadien-Copolymere oder Gemische oder Copolymere davon handelt.

20. Kompaktiertes granuliertes Additivgemisch mit reduzierter Bröckligkeit zum Zugeben zu einem Grundpolymer, umfassend:
(a) ein primäres Antioxidans in Form eines gehinderten Phenols;
(b) Erucamid;
(c) Glycerylmonostearat;
(d) Natriumbenzoat; und
(e) ein oder mehrere Kompaktierungshilfsmittel, die aus der Gruppe ausgewählt sind, die aus Phlogopit-Glimmer, Muscovit-Glimmer, Titandioxid und Gemischen davon besteht;
wobei das kompaktierte granulierte Additivgemisch keinen Polymerträger enthält.

21. Additivgemisch gemäß Anspruch 20, wobei es sich bei dem einen oder den mehreren Kompaktierungshilfsmitteln um Phlogopit-Glimmer oder Muscovit-Glimmer handelt und wobei der Phlogopit-Glimmer oder Muscovit-Glimmer etwa 0,5 bis etwa 20 Gew.-% des Additivgemischs ausmacht.

22. Additivgemisch gemäß Anspruch 21, wobei es sich bei dem einen oder den mehreren Kompaktierungshilfsmitteln um ein Gemisch aus Titandioxid in einer Menge von etwa 0,5 bis etwa 10 Gew.-% des Additivgemischs und Phlogopit-Glimmer, Muscovit-Glimmer oder Gemischen davon in einer Menge von etwa 0,5 bis etwa 10 Gew.-% des Additivgemischs handelt.

## Revendications

1. Mélange d'additifs compactés et granulés destiné à réduire la friabilité d'un polymère de base, comprenant :
(a) un mélange d'additifs de base ; et
(b) un ou plusieurs auxiliaires de compaction sélectionnés dans le groupe composé de silicates métalliques, de nano-argile montmorillonite, de silice, de carbonate de calcium, de sulfate de baryum, de dioxyde de titane, d'oxyde de zinc, et de mélanges de ceux-ci, où le ou les auxiliaires de compaction ne fondent pas en liquide dans l'environnement d'un compacteur, et où le mélange d'additifs compactés et granulés ne contient pas de support de polymère.

2. Mélange d'additifs selon la revendication 1, dans lequel le mélange d'additifs de base comprend un ou plusieurs antioxydants phénoliques, stabilisants de traitement, agents de neutralisation de l'acide, stabilisants à la lumière UV, absorbeurs de lumière UV, agents antistatiques, inhibiteurs de la catalyse métallique, agents glissants, agents anti-adhérents, agents de nucléation, lubrifiants, agents de démoulage, agents ignifugeants ou mélanges de ceux-ci.

3. Mélange d'additifs selon la revendication 2, dans lequel le mélange d'additifs de base comprend un ou plusieurs antioxydants phénoliques, et dans lequel les antioxydants phénoliques sont des antioxydants primaires phénoliques encombrés.

4. Mélange d'additifs selon la revendication 2, dans lequel le mélange d'additifs de base comprend un ou plusieurs stabilisants de traitement, et dans lequel les stabilisants de traitement sont des phosphites, des phosphonites, ou des mélanges de ceux-ci.

5. Mélange d'additifs selon la revendication 2, dans lequel le mélange d'additifs de base comprend un ou plusieurs agents de neutralisation de l'acide, et dans lequel les agents de neutralisation de l'acide sont des stéarates métalliques, des hydroxyl-carbonates d'aluminium et de magnésium hydratés, de l'oxyde de zinc, ou des mélanges de ceux-ci.

6. Mélange d'additifs selon la revendication 2, dans lequel le mélange d'additifs de base comprend un ou plusieurs stabilisants à la lumière UV, et dans lequel les stabilisants à la lumière UV sont des stabilisants à la lumière de type amine encombrée.

7. Mélange d'additifs selon la revendication 2, dans lequel le mélange d'additifs de base comprend un ou plusieurs absorbeurs de lumière UV, et dans lequel les absorbeurs de lumière UV sont des triazines, des benzophénones, des benzotriazoles, des hydroxybenzoates, ou des mélanges de ceux-ci.

8. Mélange d'additifs selon la revendication 2, dans lequel le mélange d'additifs de base comprend un ou plusieurs agents antistatiques, et dans lequel les agents antistatiques sont le monostéarate de glycéryle (GMS), des amines éthoxylées, ou des mélanges de ceux-ci.

9. Mélange d'additifs selon la revendication 2, dans lequel le mélange d'additifs de base comprend un ou plusieurs inhibiteurs de la catalyse métallique, et dans lequel les inhibiteurs de la catalyse métallique sont des propionohydrazides.

10. Mélange d'additifs selon la revendication 2, dans lequel le mélange d'additifs de base comprend un ou plusieurs agents glissants, et dans lequel les agents glissants sont de l'érucamide, de l'oléamide, du béhénamide, de l'érucamide d'érucyle ou des mélanges de ceux-ci.

11. Mélange d'additifs selon la revendication 2, dans lequel le mélange d'additifs de base comprend un ou plusieurs agents anti-adhérents, et dans lequel les agents anti-adhérents sont de la silice synthétique, de la silice naturelle, de l'aluminosilicate de sodium et de calcium, ou des mélanges de ceux-ci.

12. Mélange d'additifs selon la revendication 2, dans lequel le mélange d'additifs de base comprend un ou plusieurs agents de nucléation, et dans lequel les agents de nucléation sont du benzoate de sodium, des acétals de sorbitol, des phosphates aromatiques sodiques, de l'adipate de sodium, ou des mélanges de ceux-ci.

13. Mélange d'additifs selon la revendication 1, dans lequel le ou les auxiliaires de compaction sont des silicates métalliques, et dans lequel les silicates métalliques sont des aluminosilicates de potassium et de magnésium, des aluminosilicates de sodium et de calcium, des silicates d'aluminium anhydres, des aluminosilicates de sodium et de potassium, des silicates de calcium, des silicates de magnésium hydratés, des aluminosilicates de sodium, un fluorosilicate synthétique de magnésium, sodium et lithium, un fluorosilicate phosphaté synthétique de magnésium, sodium et lithium, ou des mélanges de ceux-ci.

14. Mélange d'additifs selon la revendication 13, dans lequel les silicates métalliques sont des aluminosilicates de potassium et de magnésium, et dans lequel les aluminosilicates de potassium et de magnésium sont du mica phlogopite, du mica muscovite, ou des mélanges de ceux-ci.

15. Mélange d'additifs selon la revendication 13, dans lequel les silicates métalliques sont du mica phlogopite ou du mica muscovite, et dans lequel le mica phlogopite ou le mica muscovite représente environ 15 à environ 20 % en poids du mélange d'additifs.

16. Mélange d'additifs selon la revendication 1, dans lequel le ou les auxiliaires de compaction sont sélectionnés parmi
(a) de l'argile montmorillonite, et dans lequel l'argile montmorillonite est modifiée avec un sel d'ammonium quaternaire,
(b) du dioxyde de titane, et dans lequel le dioxyde de titane est enrobé avec un stéarate métallique,
(c) des aluminosilicates de potassium et de magnésium et du dioxyde de titane, et dans lequel les aluminosilicates de potassium et de magnésium sont du mica phlogopite, du mica muscovite, ou un mélange de ceux-ci, ou
(d) des silices amorphes synthétiques.

17. Mélange d'additifs selon la revendication 1, dans lequel le mélange d'additifs de base a une température de fusion inférieure à environ 90 °C.

18. Procédé de préparation d'une résine polymère, comprenant les étapes consistant à :
chauffer un polymère de base pour produire un flux de polymère fondu ;
ajouter le mélange d'additifs compactés et granulés de la revendication 1 au flux de polymère fondu pour produire un flux polymère avec additif, où le mélange d'additifs compactés et granulés est exempt de support de polymère ; et
laisser le flux de polymère avec additif refroidir et durcir pour produire une résine polymère.

19. Procédé selon la revendication 18, dans lequel le polymère de base est du polyéthylène, du polypropylène, des copolymères d'éthylène-propylène, des copolymères d'éthylène-alphaoléfine, du polybutène-1, du polystyrène, des copolymères d'éthylène-acétate de vinyle, des copolymères d'éthylène-alcool de vinyle, des copolymères de styrène-butadiène, et des mélanges et copolymères de ceux-ci.

20. Mélange d'additifs compactés et granulés présentant une friabilité réduite, à ajouter à un polymère de base, et comprenant :
(a) un antioxydant primaire phénolique encombré ;
(b) de l'érucamide ;
(c) du monostéarate de glycéryle ;
(d) du benzoate de sodium ; et
(e) un ou plusieurs auxiliaires de compaction sélectionnés dans le groupe composé du mica phlogopite, du mica muscovite, du dioxyde de titane et de mélanges de ceux-ci, où le mélange d'additifs compactés et granulés ne contient pas de support de polymère.

21. Mélange d'additifs selon la revendication 20, dans lequel le ou les auxiliaires de compaction sont du mica phlogopite ou du mica muscovite, et dans lequel le mica phlogopite ou le mica muscovite représente environ 0,5 à environ 20 % en poids du mélange d'additifs.

22. Mélange d'additifs selon la revendication 21, dans lequel le ou les auxiliaires de compaction sont un mélange de dioxyde de titane, à raison d'environ 0,5 % à environ 10 % en poids du mélange d'additifs, et du mica phlogopite, du mica muscovite ou des mélanges de ceux-ci, à raison d'environ 0,5 % à environ 10 % en poids du mélange d'additifs.
